# EUROPEAN PATENT APPLICATION

(11) **EP 4 104 922 A1**
(43) Date of publication of application: **21.12.2022**
(21) Application number: 21204944.9
(22) Date of filing: 27.10.2021
(51) Int. Cl.: B01J 19/00, B01J 19/24, C07C 263/10

(54) **REACTOR AND PROCESS FOR PREPARING ISOCYANATES**

(30) Priority: 14.06.2021 US 202163210145 P; 05.07.2021 EP 21183607
(71) Applicant: Covestro Deutschland AG, 51373 Leverkusen (DE)
(72) Inventor: The designation of the inventor has not yet been filed
(74) Representative: Levpat

(57) **Abstract**

The invention relates to a tubular reactor for gas phase phosgenation of at least one organic amine comprising
• at least one inlet device for starting materials,
• an outer shell with inner diameters Dri,
• a tubular inner sleeve disposed within a segment of the outer shell, having a length Lₛ along its longitudinal axis and outer diameters Dₛₒ, wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap between the inner sleeve and the outer shell, and wherein the inlet device is disposed within the outer shell

## Description

### TECHNICAL FIELD

The invention relates to a tubular reactor for gas phase phosgenation of at least one organic amine, to a process for producing isocyanates by phosgenation of organic amines utilizing such a reactor, and to a computer implemented method for optimizing the design of a tubular reactor for gas phase phosgenation of at least one organic amine.

### BACKGROUND OF THE INVENTION

Isocyanates are prepared in large quantities and are used mainly as starting materials for the preparation of polyurethanes. They are usually prepared by converting the appropriate amines with a stoichiometric excess of phosgene. The phosgenation of the amines can take place in the gas phase with excess phosgene, the latter being normally recovered together with the gaseous hydrogen chloride by-product that is liberated in the course of the reaction. By-products of the reaction can form solid deposits on the reactor walls, which are one of the main causes for limited operation times of the continuous gas phase phosgenation process e.g. due to reactor maintenance downtimes. Despite all efforts to minimize the formation of deposits, this remains a challenge in gas phase phosgenation reactions.

EP1319655 A2 and EP1449826 A1 describe the use of an inliner inside the reactor in order to minimize the duration of maintenance shutdowns caused by such solid deposits and allowing a faster restart of the reactor after exchange of the crusted inliner.

In EP1449826 A1 a tubular reactor is disclosed that is fixed inside a reactor case and that receives vaporous amine through a multitude of nozzles directed parallel to the tube reactor while the phosgene stream is fed through the remaining free space between the nozzles. However, both documents are silent with regard to the ratio of the inliner diameter to the reactor case diameter or the width of the gap that is formed between those two.

WO2014006164 A1 discloses a reactor for carrying out exothermic reactions in general which comprises an inner shell and wherein the inner shell has a distance of at least 50 mm from the inside of the outer reactor wall. The inner shell and the gap formed between the inner shell and the outer wall provide a layer of insulation, thereby preventing excessive temperatures at the reactor wall which could otherwise lead to embrittlement of the reactor wall material. However, it has been found by the inventors of the present invention that for the gas phase phosgenation of amines a distance of at least 50 mm between the inner shell and the reactor wall is not a sufficient parameter to suppress the formation of solid deposits inside the reactor.

It is an objective of the present invention to provide a reactor for the phosgenation of amines that allows isocyanate synthesis in the gas phase wherein the formation of solid deposits is reduced so that the continuous phosgenation operation can be operated with less frequent process interruptions.

### BRIEF DESCRIPTION OF THE FIGURES

The present invention will now be described for purposes of illustration and not limitation in conjunction with the figure, wherein:

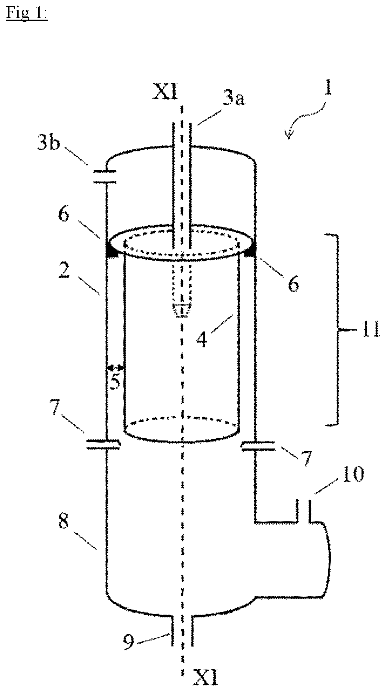

FIG 1 shows a schematic of a tubular reactor with an inner sleeve, wherein the dashed line XI-XI represents the longitudinal axis of the inner sleeve.

### SUMMARY OF THE INVENTION

The present invention relates to a tubular reactor for gas phase phosgenation of at least one organic amine comprising
- at least one inlet device for starting materials,
- an outer shell with inner diameters Dᵣᵢ,
- a tubular inner sleeve disposed within a segment of the outer shell, having a length Lₛ along its longitudinal axis and outer diameters Dₛₒ,
wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap between the inner sleeve and the outer shell, and wherein the inlet device is disposed within the outer shell.

Furthermore, the present invention pertains to a process for the production of an isocyanate by phosgenation of at least one organic amine, comprising the steps of
(i) providing a gaseous stream of the amine;
(ii) providing a gaseous stream of phosgene;
(iii) mixing the amine stream from step (i) and the phosgene stream from step (ii) and passing the resulting mixture through a reaction zone to conduct a reaction of the amine with excess phosgene and obtain a gaseous reaction product mixture;
(iv) cooling the reaction product mixture obtained in step (iii) by contacting it with a quench liquid in a quench zone to obtain a quenched product mixture; and
(v) separating the isocyanate from the quenched product mixture obtained in step (iv);
characterized in that the reaction in step (iii) is carried out in the tubular reactor of the present invention.

### DETAILED DESCRIPTION OF THE INVENTION

For purposes of the following detailed description, it is to be understood that, other than in any operating examples, or where otherwise indicated, all numbers expressing, for example, quantities of ingredients used in the specification and claims the respective number includes "about" the respective number. For example, if the number 10 is described "10" as well as "about 10" is included.

In the present invention, any numerical ranges that are specified in the format "from x to y" include the specified values. If several preferred numeric areas are specified in this format, it is natural that all areas created by combining the different endpoints are also captured.

According to the invention, the references to "comprising", "containing" etc. preferably mean "essentially consisting of" and very particularly preferably "consisting of". The further embodiments mentioned in the patent claims, in the aspects and in the description can be combined as desired, provided that the opposite is not clearly apparent from the context.

In the context of the invention, each pair of Dᵣᵢ and Dₛₒ are determined as follows:
The inner diameter Dᵣᵢ of the outer shell equals the length of the shortest straight line segment,
wherein this shortest straight line segment
   - lies within a cross section perpendicular to the longitudinal axis of the inner sleeve,
   - passes through the longitudinal axis of the sleeve, and
   - has its endpoints on the inner wall of the outer shell,
whereas the corresponding outer diameter Dₛₒ of the inner sleeve equals the length of a straight line segment, wherein this straight line segment
   - lies within the same cross section as Dᵣᵢ,
   - passes through this longitudinal axis of the sleeve,
   - has its endpoints on the outer wall of the inner sleeve, and
   - has the same orientation within said cross section as Dᵣᵢ.
In the case that within the same cross section more than one shortest straight line segments of the same length exist that have different orientations, the orientation of Dᵣᵢ and consequently the orientation of Dₛₒ shall be the orientation that leads to the highest value for Dₛₒ.

If still more than one orientation exists that results in the same lowest values for Dᵣᵢ and the same highest values for Dₛₒ, respectively, which may happen in the case of very regular reactor geometries (e.g. in cylindrical reactors), those remaining orientations are to be considered equivalent and any of those remaining orientations can be selected in order to define Dᵣᵢ and Dₛₒ and calculate their ratio R_{d}.

Following these rules for the determination of Dᵣᵢ and Dₛₒ, these diameters can adopt different values along the reactor, because both, the inner sleeve and the outer shell of the reactor may undergo extensions or constrictions, or they may have irregularities like indentations, shape changes or the like.

FIG 1 shows a schematic of a tubular reactor (1) for gas phase phosgenation of an organic amine, wherein vertical dashed line XI-XI represents the longitudinal axis of the inner sleeve.

The tubular reactor (1) consists of an outer shell (2) with inner diameters Dᵣᵢ and two inlet devices (3a) and (3b), one for phosgene and one for at least one organic amine, respectively. Optionally an inert gas can be fed to the reactor together with the phosgene and/or the at least one organic amine. In the reactor (1) a tubular inner sleeve (4) with a length Lₛ along its longitudinal axis (XI-XI) and outer diameters Dₛₒ is arranged in a way that a gap (5) for a gaseous material exists between the outer shell (2) and the inner sleeve (4). The sleeve rests on a support ring (6) attached to the inner wall of the outer shell (2). The length Lₛ of the inner sleeve (4) reaches from top of the sleeve near the inlet devices (3) to the bottom near the quench nozzles (7) in the direction of its longitudinal axis. Downstream of the quench nozzles (7) the reactor comprises a collection tank (8) with an outlet (9) for the liquid reaction products and an outlet (10) for the gaseous parts of the reaction product. The section of the outer shell through which the inner sleeve extends is indicated by the curly brace (11).

The section (11) of the outer shell (2) through which the inner sleeve extends has the same length as the length Lₛ of the inner sleeve (4) and for any given cross section of the reactor along said section, the diameters Dₛₒ and Dᵣᵢ can be derived. A reactor having a gap (5) for a gaseous material between the inner sleeve (4) and the outer shell (2), and for which Dₛₒ is in a ratio R_{d} to the inner diameter Dᵣᵢ from 0.600 to 0.990, calculated by the following equation R_{d} = Dₛₒ/Dᵣᵢ, over at least 50 % of the length Ls of the inner sleeve, is a reactor according to the invention.

During operation of the tubular reactor (1), at least one organic amine vapor, phosgene vapor and optionally an inert gas are fed through the inlets (3a) and (3b) with the reaction gas flowing from top to bottom through the inside of the inner sleeve (4). The gap (5) is in fluidic connection with the inside of the inner sleeve so that small amounts of the reaction gas can enter and exit the gap for pressure equalization and improved temperature adjustment of the sleeve. After exiting from the space inside the inner sleeve, the reaction mixture is rapidly cooled down, preferably by quenching it with a liquid solvent spray via the quench nozzles (7). Upon this cooling, the reaction mixture is at least partly condensed and enters the collection tank (8). Here, non-condensed gases are separated from the liquid and optionally solid parts of the reaction mixture. The liquid and optionally solid parts of the reaction mixture in the collection tank (8) exit via the outlet (9) for liquid reaction products. The non-condensed gas stream exiting via the outlet (10) is usually separated in subsequent steps in order to recover unreacted phosgene and HCl while the liquid stream exiting via the outlet (9) is usually worked up in subsequent steps in order to isolate the formed isocyanate as the desired reaction product and recover solvents and/or phosgene.

The present invention in particular pertains to the following aspects:
1. A tubular reactor (1) for gas phase phosgenation of at least one organic amine comprising
   - at least one inlet device (3a) for starting materials,
   - an outer shell (2) with inner diameters Dᵣᵢ
   - a tubular inner sleeve (4) disposed within a segment of the outer shell, having a length Lₛ along its longitudinal axis and outer diameters Dₛₒ,
   wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap (5) between the tubular inner sleeve (4) and the outer shell (2), and wherein the inlet device (3a) is disposed within the outer shell (2).
2. The reactor according to aspect 1 wherein R_{d} is in the range from 0.600 to 0.990 along at least 70 % of the length Lₛ of the inner sleeve (4), preferably along at least 90 % of the length Lₛ of the inner sleeve (4), more preferably along at least 95 % of the length Lₛ of the inner sleeve (4) and even more preferably along at least 99 % of the length Lₛ of the inner sleeve (4).
3. The reactor according to aspect 1 or 2 wherein the inlet device (3a) for the amine comprises an annular gap nozzle.
4. The reactor according to any of aspects 1 to 2 wherein the gap (5) between the inner sleeve and the outer shell (2) has a width that does not deviate more than 30 %, preferably not more than 10 % and more preferably not more than 5 % from its narrowest width within a given cross section perpendicular to the longitudinal axis of the inner sleeve (4).
5. The reactor according to any of aspects 1 to 4 wherein at least the inner sleeve (4), preferably the inner sleeve (4) and the segment of the outer shell through which the inner sleeve extends (11), are rotationally symmetric at 120° or at 180°.
6. The reactor according to any of aspects 1 to 5 wherein at least the inner sleeve (4), preferably the inner sleeve (4) and the segment of the outer shell through which the inner sleeve extends (11) are circularly symmetric.
7. The reactor according to any of aspects 1 to 6 wherein over at least 90% of the cross sectional areas of the outer shell (2) perpendicular to its longitudinal axis within the segment through which the inner sleeve extends (11) are in the range from 75 cm² to 18000 cm², preferably from 175 cm² to 5000 cm², more preferably from 300 cm² to 3000 cm².
8. The reactor according to any of aspects 1 to 7 wherein the inner sleeve (4) is of a cylindrical shape over at least 70 % of its length along the gas flow path.
9. The reactor according to any of aspects 1 to 8 wherein the reactor (1) is arranged upright with the longitudinal axis preferably being inclined from the vertical at an angle of 10° or less, more preferably 5° or less, even more preferably 2° or less and most preferably 1° or less and the at least one inlet device (3a) being located in the upper part of the reactor (1) so that the direction of gas flow is from top to bottom.
10. The reactor according to any of aspects 1 to 9 wherein the ratio R_{d} is in the range from 0.700 to 0.985, preferably in the range from 0.750 to 0.980 and more preferably in the range from 0.800 to 0.975.
11. The reactor according to any of aspects 1 to 10 wherein the outer shell (2) and the inner sleeve (4) are composed of metallic materials, preferably steel, high grade stainless steel, titanium, nickel or metal alloys, including, but not limited to nickel alloys comprising iron and chromium, or nickel alloys comprising molybdenum and chromium.
12. The reactor according to any of aspects 1 to 11 wherein the length Lₛ of the inner sleeve (4) along its longitudinal axis is from 1.5 x its largest diameter Dₛₒ to 150 x its largest Diameter Dₛₒ, preferably from 3 x its largest diameter Dₛₒ to 100 x its largest diameter Dₛₒ, even more preferably 10 x its largest diameter Dₛₒ to 70 x its largest diameter Dₛₒ and most preferably 12 x its largest diameter Dₛₒ to 50 x its largest diameter Dₛₒ.
13. The reactor according to any of aspects 1 to 12 wherein the width of the gap (5) that exists between the inner sleeve (4) and the outer shell (2) is in the range from 1 to less than 50 mm, preferably 1 to 49 mm, more preferably 2 to 48 mm, even more preferably 4 to 45 and most preferably 8 to 40 mm.
14. The reactor according to any of aspects 1 to 13 wherein the inner sleeve (4) flares out conically at its lower end so that at the very end of the inner sleeve, the outer diameter Dₛₒ of the sleeve is 4 to 40 mm, preferably 6 to 20 mm and more preferably 6 to 10 mm smaller than the inner diameter Dᵣᵢ of the outer shell (2) at that very end of the inner sleeve.
15. The reactor according to any of aspects 1 to 14 further comprising one or more quench nozzles (7) and wherein the length L_{S} of the inner sleeve (4) reaches from top of the sleeve near the inlet devices (3) to the bottom near the quench nozzles (7) in the direction of its longitudinal axis.
16. The reactor according to any of aspects 1 to 15 further comprising downstream of the quench nozzles (7) a collection tank (8) with an outlet (9) for the liquid reaction products and an outlet (10) for the gaseous parts of the reaction product.
17. A process for the production of an isocyanate by phosgenation of at least one organic amine, comprising the steps of
   (i) providing a gaseous stream of the amine ;
   (ii) providing a gaseous stream of phosgene;
   (iii) mixing the amine stream from step (i) and the phosgene stream from step (ii) and passing the resulting mixture through a reaction zone to conduct a reaction of the amine with excess phosgene and obtain a gaseous reaction product mixture;
   (iv) cooling the reaction product mixture obtained in step (iii) by contacting it with a quench liquid in a quench zone to obtain a quenched product mixture; and
   (v) separating the isocyanate from the quenched product mixture obtained in step (iv)
   characterized in that the reaction in step (iii) is carried out in a reactor (1) according to any of aspects 1 to 16.
18. The process according to aspect 17 comprising the additional steps of
   (vi) shutting down the phosgenation;
   (vii) removing the inner sleeve (4) from the reactor;
   (viii) inserting a new or cleaned inner sleeve (4) into the reactor; and
   (ix) restarting the phosgenation.
19. The process according to aspect 17 or 18 wherein before the phosgenation is started or restarted, the steps of
   (x) defining the type of organic amine to be phosgenated and the desired throughput for the following production run and providing this information;
   (xi) analyzing the information provided in step (x) to determine a sleeve size and a nozzle position to be used; and
   (xii) mounting the sleeve (4) and the amine nozzle as determined in step (xi) into the tubular reactor;
   are carried out.
20. The process according to any of aspect 17 to 19 wherein the reaction mixture is rapidly cooled down after exiting from the space inside the inner sleeve (4), preferably by quenching the reaction mixture with a liquid solvent spray via the quench nozzles (7).
21. The process according to any of aspect 17 to 20 wherein the organic amine is selected from the group consisting of hexamethylene diamine, pentamethylene diamine and 1-amino-3,5,5-trimethyl-5-aminomethylcyclohexane.
22. A computer implemented method for optimizing the design of a tubular reactor (1) for gas phase phosgenation of at least one organic amine, the method comprising the steps of:
   a. defining a desired residence time for the reaction mixture in the reaction zone of the reactor;
   b. calculating a theoretical residence time from the reactor geometry and the envisaged process conditions including temperature, pressure and feed streams of phosgene, amine and optionally inert gas;
   c. comparing the desired residence time and the calculated theoretical residence time;
   d. adjusting the reactor design until the calculated theoretical residence time falls within the range from 50% to 200%, preferably 70% to 150%, of the desired residence time.
   e. Selecting a nozzle position and/or a sleeve from the adjusted reactor design for assembling the tubular reactor.
   wherein the assembled tubular reactor (1) comprises
   - at least one inlet device (3a) for starting materials,
   - an outer shell (2) with inner diameters Dᵣᵢ,
   - a tubular inner sleeve (4) disposed within a segment of the outer shell, having a length Lₛ along its longitudinal axis and outer diameters Dₛₒ,
   and wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap (5) between the inner sleeve and the outer shell (2), and wherein the inlet device (3) is disposed within the outer shell (2).

It was found that a ratio R_{d} from 0.600 to 0.990 can reduce the formation of solid deposits during the gas phase phosgenation of amines, and in particular di- or polyamines. When using reactors with a dimension ratio R_{d} outside these boundaries, faster formation of deposits was observed. Without wishing to be bound by theory, the underlying causes for the formation of deposits appear to be different for diameter ratios above 0.990 compared to diameter ratios R_{d} below 0.600. The inventors have found indications that for ratios R_{d} above 0.990 cold spots may form on the inner sleeve, leading to the formation of deposits and, consequently to a deflection of the gas stream while still inside the inner sleeve. Furthermore, reactors with such a high ratio R_{d} require high precision during fabrication of the parts as well as during mounting and dismounting of the inner sleeve. For ratios R_{d} below 0.600, the cause seems to be different as deposits are mainly observed downstream of the inner sleeve. The deposits formed in gas phase phosgenation reactors typically contain carbamoyl chlorides, amine hydrochlorides, isocyanurate trimers or oligomers, ureas, carbodiimides or simply thermal decomposition products.

To achieve the effects of the invention, R_{d} shall be within the range according to the invention along at least 50 % of the length Lₛ of the inner sleeve, preferably along at least 70 % of the length Lₛ of the inner sleeve, more preferably along at least 90 % of the length Lₛ of the inner sleeve, even more preferably along at least 95 % of the length Lₛ of the inner sleeve, and most preferably along at least 99 % of the length Lₛ of the inner sleeve.

Tubular reactors that are suitable for the gas phase phosgenation of amines are known from the state of the art. Preferably, they do not contain any moving parts within the reaction space, which is defined as the space inside the reactor, where the reaction takes place. Typically, these reactors are rotationally symmetric, preferably circularly symmetric tubular reactors. Rotationally symmetric bodies (rotational symmetry is sometimes also referred to as radial symmetry) are bodies which look the same after a specific rotation of e.g. 60° (6-fold rotational symmetry), 90° (4-fold rotational symmetry), 120° (3-fold rotational symmetry), or 180° (2-fold rotational symmetry) around their central axis. Of course, bodies of a higher symmetry also include the corresponding lower symmetries, e.g. a body that has 6-fold symmetry also includes 3- and 2-fold symmetry and a body with 8-fold symmetry includes 4-fold and 2-fold symmetry as well. If a body is rotationally symmetric with respect to any angle of rotation around its central axis, it is a circularly symmetric body. Thus, circular symmetry is a special case of rotational symmetry.

It is known to those skilled in the art that in processing equipment there are always small deviations from the perfectly symmetric shapes caused for example by manufacturing tolerances or for example by brackets, flanges or nozzles which are for example required to attach process control equipment to the reactor. These shall not be considered to break the overall rotational symmetry of the reactor in the light of the present invention. Likewise, equipment located upstream or downstream of the reaction space shall not be considered to break the symmetry of the reactor.

The reactor (1) comprises at least one inlet device (3) for the starting materials. Preferably, the reactor (1) comprises at least one inlet device (3), more preferably exactly one inlet device (3a) for the addition of the at least one organic amine and at least one inlet device, more preferably exactly one inlet device (3b) for the addition of the phosgene. Thus, it is particularly preferred that the reactor (1) comprises no more than one inlet device (3) for each reactant. The position of the inlet device (3) through which the amine is added during operation of the reactor can be selected according to the needs of the reaction. A position further downstream in the reactor will reduce residence time of the amine in the reaction zone while a position further upstream will increase residence time. This can be a useful parameter in case of planned partial load operation or in case one reactor is used to produce different isocyanates that require different residence times. Changing the position of the organic amine's feed point allows changing the residence time without affecting too many other parameters. In the case that phosgene is added into a gaseous amine stream, the position of the phosgene inlet device (3) can be changed accordingly in order to adjust the residence time of the phosgene in the reaction zone.

In the reactor (1) according to the invention it is also possible to change the diameter of the inner sleeve (4) within the given boundaries by replacing a given inner sleeve with a different one in order to adjust residence times to the needs of the reaction. This however will also have an impact on other process parameters like gas velocities inside the reaction zone, pressure drops, mixing times and the like.

In a preferred embodiment of the reactor (1) according to the invention, the inlet device (3a) for the amine comprises an annular gap nozzle. Preferably, this annular gap nozzle has a central channel which is suitable for feeding the gaseous amine stream to the reactor and an annular channel around this central channel which is suitable for feeding an inert gas to the reactor. Preferably, the inert gas is nitrogen or solvent vapor, the solvent vapor comprising chlorobenzene and/or dichlorobenzene.

With regard to the reactor (1) of the present invention it is preferable that at least the inner sleeve (4), preferably the inner sleeve (4) and the segment of the outer shell through which the inner sleeve extends (11) are rotationally symmetric, preferably rotationally symmetric at 120° or at 180°, with regard to their respective longitudinal axes. Even more preferably, at least the inner sleeve (4), preferably the inner sleeve (4) and the segment of the outer shell through which the inner sleeve extends (11) are circularly symmetric with regard to their longitudinal axis. Within said segment (11) of the outer shell, the cross sectional area of the outer shell perpendicular to its longitudinal axis is in the range from 75 cm² to 18000 cm², preferably from 175 cm² to 5000 cm², more preferably from 300 cm² to 3000 cm².

In another preferred embodiment of the reactor (1) according to the invention, the inner sleeve (4) is of a cylindrical shape over at least 70 % of its length Lₛ. A cylindrical shape minimizes the amount of backmixing and thereby further reduces formation of solid deposits within the reaction zone.

In another preferred embodiment of the reactor (1) according to the invention, the reactor (1) is arranged upright with the longitudinal axis preferably being inclined from the vertical at an angle of 10° or less, more preferably 5° or less, even more preferably 2° or less and most preferably 1° or less. It is further preferred that the at least one inlet device (3) are located in the upper part of the reactor so that the direction of gas flow is from top to bottom. Such an arrangement prevents negative impacts of gravitational force to the gas flow and thereby minimizes the risk of plugging the reactor with solid deposits.

Methods to fix the inner sleeve in the outer shell are known to those skilled in the art. For example, it is possible to have a flange attached at the inner sleeve that extends outwards from the sleeve and can be clamped into a flange connection in the outer shell. Preferably, said flange is attached at the upper end of the sleeve. More preferably, the reactor (1) is equipped with support brackets (6) or a circumferential support beam (6) to which the sleeve can attached or on which it is resting, again preferably with a flange attached to the upper end of the sleeve (4). The inner sleeve and/or the reactor wall, preferably the inner sleeve, can be equipped with spacers in order to achieve a good centricity of the inner sleeve inside the outer reactor wall. Preferably, spacers are located on the outside of the inner sleeve. Preferably they are located in one or more levels along the lengths of the sleeve and in each of those levels there are preferably three or more, more preferably three of these spacers distributed preferably evenly around the circumference of the sleeve. Preferably, the spacers consist of an insulating material in order to avoid heat loss via the spacers.

In a preferred embodiment of the reactor (1) according to the invention, the gap (5) that exists between the inner sleeve (4) and the outer shell (2) of the reactor is sealed on one end, preferably the upstream end of the sleeve, while being open at the other end. Such an arrangement allows gas to enter and exit the gap space without leading to an actual flow through the gap (5). The gas filled gap (5) in fluidic contact with the reaction zone can provide thermal insulation and results in a number of advantages: Firstly, the isolated inner sleeve (4) will adapt faster to a steady temperature profile after starting up the reactor or changing process conditions like temperatures, phosgene excess, pressure or load for other reasons. Secondly, the insulation can prevent cold spots that may form at the interface of the inner sleeve (4) to the reaction zone and that again can be a cause for the formation of solid deposits. Such cold spots may be caused by less than perfect insulation of the outer shell (2) or by heat sinks due to flange connections or the like in the outer shell of the reactor. Thirdly, the fluidic connection enables gas to enter or exit the gap (5) between the inner sleeve and the outer wall of the reactor, thereby allowing for pressure equalization, so that the inner sleeve can be made of relatively thin material. Preferably, the thickness of the inner sleeve (4) is ≤10 mm, more preferably ≤5 mm and most preferably ≤3 mm.

In another embodiment of the reactor (1) according to the invention, the ratio R_{d} is in the range from 0.700 to 0.985, preferably in the range from 0.750 to 0.980 and more preferably in the range from 0.800 to 0.975.

Preferably, the gap (5) between the inner sleeve (4) and the outer shell (2) has a width that does not deviate more than 30 %, preferably not more than 10 % and more preferably not more than 5 % from its narrowest width within a given cross section perpendicular to the longitudinal axis of the inner sleeve (4). Also preferably, the difference between Dᵣᵢ and Dₛₒ does not deviate more than 30 %, from its smallest number along length Lₛ. In the present invention, the gap width is to be measured in radial direction from the longitudinal axis of the inner sleeve. It is also preferred that the width of the gap (5) that exists between the inner sleeve (4) and the outer shell (2) is in the range from 1 to less than 50 mm, preferably 1 to 49 mm, more preferably 2 to 48 mm, even more preferably 4 to 45 and most preferably 8 to 40 mm. In case the gap (5) is wider than 4 mm, it is preferred to use a sleeve (4) that flares out conically at its lower end so that at the very end of the inner sleeve, the outer diameter Dₛₒ of the sleeve is 4 to 40 mm, preferably 6 to 20 mm and more preferably 6 to 10 mm smaller than the corresponding inner diameter Dᵣᵢ at that position. This allows for a smoother transition and reduces circulation and backmixing at the outlet of the inner sleeve (4).

The lower end of the sleeve is preferably located at or upstream of a quench zone, preferably upstream of a quench zone, so that the reaction gas that exits from the inner sleeve is passed directly into said quench zone for rapid cooling. The quench is equipped with inlets (7) for a cooling liquid. Preferably, the quench comprises spray nozzles (7) through which the cooling liquid can be sprayed into the quench zone in order to rapidly cool down the hot reaction gas mixture. Downstream of the quench zone, there is usually a collection tank (8) to collect the effluent of the reactor (1). This collection tank can also be used to separate condensed liquid parts of the reaction mixture from the remaining gases and transfer those separately into the respective workup sections of the production unit.

In a further embodiment of the reactor (1) according to the invention, the outer shell (2) and the inner sleeve (4) are preferably composed of metallic materials. Preferred metallic materials are steel, high grade stainless steel, like for example types 1.4571, 1.4401 or 1.4404, titanium, nickel or metal alloys, including, but not limited to nickel alloys comprising iron and chromium (Inconel), or nickel alloys comprising molybdenum and chromium (Hastelloy). It is also possible for the outer shell (2) of the reactor and/or the inner sleeve (4) to be constructed of combinations of the above-mentioned materials. For example, in areas with high temperatures, metals with the highest temperature and corrosion stability can be used, while in areas with lower temperatures, lower grade material can be used. Particularly preferred metallic materials are nickel alloys comprising iron and chromium or nickel alloys comprising molybdenum and chromium. The inner sleeve (4) can be replaced from time to time and does not need to achieve the same lifetime as the outer shell (2) of the reactor. Based on economic considerations, it is therefore also preferably made from a lower grade material than the outer shell of the reactor. Most preferably the inner sleeve is made from high grade stainless steel.

Preferably, the length Lₛ of the inner sleeve (4) along its longitudinal axis is from 1.5 x its largest diameter Dₛₒ to 150 x its largest Diameter Dₛₒ, preferably from 3 x its largest diameter Dₛₒ to 100 x its largest diameter Dₛₒ, even more preferably 10 x its largest diameter Dₛₒ to 70 x its largest diameter Dₛₒ and most preferably 12 x its largest diameter Dₛₒ to 50 x its largest diameter Dₛₒ.

The present invention also provides a process for the preparation of isocyanates by converting amines with phosgene in the gas phase, wherein the conversion is carried out in a reactor (1) according to the invention. The advantage of the process according to the invention lies in particular in longer reaction times before a maintenance shutdown is necessary, while at the same time allowing short maintenance shutdown due to the use of an inner sleeve in the reactor (1) according to the invention.

The preparation of isocyanates by converting amines with phosgene in the gas phase is known from the state of the art. For example, such processes are described in EP0289840 A1, EP1319655 A2, EP1555258 A1, EP144826 A1, or recently in WO2019145380 A1.

The reactor (1) according to the present invention is suitable for the phosgenation of organic amines, in particular organic amines having primary amino groups. Preference is given to those compounds having at least 2, particularly preferably 2 or 3 NH₂ groups which may be bonded to an aliphatic, cycloaliphatic, araliphatic or aromatic carbohydrate backbone. In accordance with the invention, very particular preference is given to those amines having 2 such NH₂-groups. Examples and further preferred organic amines are the same as listed below.

Furthermore, the invention relates to a process for the production of an isocyanate by phosgenation of at least one organic amine, comprising the steps of
(i) providing a gaseous stream of the amine;
(ii) providing a gaseous stream of phosgene;
(iii) mixing the amine stream from step (i) and the phosgene stream from step (ii) and passing the resulting mixture through a reaction zone to conduct a reaction of the amine with excess phosgene and obtain a gaseous reaction product mixture;
(iv) cooling the reaction product mixture obtained in step (iii) by contacting it with a quench liquid in a quench zone to obtain a quenched product mixture; and
(v) separating the isocyanate from the quenched product mixture obtained in step (iv);
characterized in that the reaction in step (iii) is carried out in a tubular reactor (1) according to the present invention.

In one embodiment of the process of the invention, the organic amine is, for example, selected from the group consisting of 2,4-diaminotoluene, 2,6-diaminotoluene, 1,4-diaminobutane, 1,5-diaminopentane (PDA), 1,6-diaminohexane (HDA), 1,11-diaminoundecane, 1-amino-3,5,5-trimethyl-5-aminomethylcyclohexane (IPDA), bis(p-aminocyclohexyl)methane (PACM), 1,5-diamino-2-methylpentane, 2,5-diamino-2,5-dimethylhexane, 1,4-diaminocyclohexane, 2,4-hexahydrotoluylenediamine, 2,6-hexahydrotoluylenediamine (H6TDA), 1,3-bis(aminomethyl)benzene (m-XDA), 1,4-bis(aminomethyl)benzene (p-XDA), isomers of bis(aminomethyl)cyclohexane (H6XDA), isomeren of bis(aminomethyl)norbornane (NBDA), neopentanediamine, 2,4,4-trimethylhexamethylenediamine, 2,2,4-trimethylhexamethylenediamine and any desired mixture thereof.

Preferred organic amines for the phosgenation in a reactor (1) according to the present invention are selected from the group consisting of 2,4-diaminotoluene, 2,6-diaminotoluene, 1,5-diaminopentane (PDA), 1,6-diaminohexane (HDA), 1-amino-3,5,5-trimethyl-5-aminomethylcyclohexane (IPDA) and any desired mixture thereof.

Particularly preferred organic amines for the phosgenation in a reactor (1) according to the present invention are selected from the group consisting of 1,5-diaminopentane (PDA), 1,6-diaminohexane (HDA), and 1-amino-3,5,5-trimethyl-5-aminomethylcyclohexane (IPDA) and any desired mixtures thereof.

Equally preferred, the organic amine is a mixture of 2,4-diaminotoluene and 2,6-diaminotoluene.

In a further embodiment of the process according to the invention, all starting materials entering the phosgenation reactor (1) have a water content of not more than 2000 ppm, preferably not more than 1000 ppm, even more preferably not more than 500 ppm by weight and most preferably not more than 300 ppm by weight.

In a further embodiment of the process according to the invention, the starting amine is evaporated and heated to a temperature of 200 to 600 °C, preferably to 220 to 500 °C and more preferably at 230 to 320 °C and most preferably to 270 to 310 °C and then fed to the reactor (1). Optionally, the amine can be mixed with an inert substance such as nitrogen or the vapors of an inert solvent, for example chlorobenzene or dichlorobenzene. The abovementioned temperatures, in case of using mixtures with inert substances, are applicable to the entire stream comprising the amine and the inert substance.

The phosgene used in the phosgenation reaction is likewise heated to a temperature of 200 to 600 °C, preferably to 220 to 500 °C and more preferably at 230 to 320 °C and most preferably to 270 to 310 °C and then fed to the reactor (1). Optionally, it can also be diluted with an inert substance, as described for the amine. The abovementioned temperatures, in case of using mixtures with inert substances, are applicable to the entire stream comprising phosgene and the inert substance.

In a further embodiment of the process according to the invention the amine stream is fed to the reactor (1) via a feed tube equipped with a smooth jet nozzle in a coaxial arrangement, while the phosgene stream is fed to the reactor (1) in a way that it passes through the annular space formed around the nozzle. In the cylindrical space downstream of the nozzle that is confined by the inner sleeve of the reactor (1), the two streams are rapidly mixed and the reaction occurs.

In general, the position of the nozzle can be selected according to the needs of the reaction. A position further downstream will reduce residence time of the amine in the reaction zone while a position further upstream will increase residence time. This can be a useful parameter in case of partial load operation or in case one reactor (1) is used to produce different isocyanates that require different residence times.

In a further embodiment of the process according to the invention, an annular gap nozzle is mounted on the amine feed tube, said annular gap nozzle having an inner channel through which the amine is fed to the reactor (1) and an annular channel surrounding the inner channel through which an inert gas stream, preferably, a Nitrogen stream is fed to the reactor (1). Such an arrangement reduces formation of deposits at the nozzle outlet. Preferably, the nitrogen stream is heated to a temperature of 200 to 600 °C, preferably to 220 to 500 °C and more preferably at 230 to 320 °C and most preferably to 270 to 310 °C before being fed to the annular gap nozzle.

The reaction takes place in a reaction zone which is located inside the inner sleeve (4). Preferably the reaction is conducted such that the amine is fully converted before entry into a quench zone which is located downstream of the inner sleeve. In said quench zone, rapid cooling is performed, preferably by contacting the hot reaction gas with a quench liquid. Suitable quench liquids are organic solvents or mixtures of such organic solvents and the isocyanate to be prepared in the process. Preferably, mixtures of an organic solvent and the isocyanate to be prepared are used as quench liquid. The solvent is preferably selected from the group consisting of chlorobenzene, o-dichlorobenzene, p-dichlorobenzene, toluene, isomers of xylene and mixtures of the aforementioned solvents. Particularly preferred solvents are chlorobenzene and o-dichlorobenzene.

Options for the construction and operation of a quench zone are known in principle from the prior art. The apparatuses and methods of the prior art can also be used in the context of the present invention. Possible configurations of the quench zone are disclosed, for example, in EP 1403248 A1 and EP 1935875 A1.

In the quench zone, the reaction products are at least partly condensed and dissolved into the quench liquid which is separated from the gaseous components. This crude liquid product is then preferably worked up by commonly known methods including distillation steps in a series of rectification towers.

In case e.g. maintenance work is required, the reactor (1) according to the invention has the advantage that the inner sleeve can easily be removed from the reactor during a maintenance shutdown for either cleaning or replacement with a new inner sleeve. Therefore, in a further embodiment of the process according to the invention, the process comprises the additional steps of
(vi) shutting down the phosgenation;
(vii) removing the inner sleeve from the reactor (1);
(viii) insert a new or cleaned inner sleeve into the reactor (1); and
(ix) restarting the phosgenation.

The reactor (1) according to the invention is highly versatile and can be used for the production of different types of isocyanates or at different throughputs. In the case that the type of the amine or the throughput is changed from one run to another, it is advisable to insert a sleeve (4) that has a tailored size for these process conditions and/or adjust the position of the amine nozzle of the inlet device (3a) in a way that allows the optimal residence time of the reaction mixture in the reaction zone. If not known, the optimal conditions can be derived from a series of tests with different combinations of inner sleeve and/or nozzle position. Typically, the residence time required to react the amine groups with the phosgene to form isocyanate is between 0.01 and 15 seconds, preferably between 0.02 and 2 seconds, depending on the type of amine used, the starting temperature, the adiabatic temperature rise, the molar ratio of amine used to phosgene, any dilution of the reactants with inert gases, and the chosen reaction pressure. It is known to the skilled artisan that for example aromatic amines require higher residence times than aliphatic amines due to their lower reactivity towards phosgene.

Therefore, in a further embodiment of the process according to the invention, before the phosgenation is started, the steps of
(x) defining the type of organic amine to be phosgenated and the desired throughput for the following production run and providing this information;
(xi) analyzing the information provided in step (x) to determine a sleeve size and a nozzle position to be used; and
(xii) mounting the sleeve and the amine nozzle as determined in step (xi) into the tubular reactor (1);
are carried out.

As mentioned before, the tubular reactor (1) can be optimized in terms of ideal residence time for a specific organic amine preferably by adjusting the nozzle position and/or sleeve design. To allow efficient and optimized design of the reactor (1) it is especially suitable to use information technology support for determination of such a reactor layout.

Thus the invention further relates to a computer implemented method for optimizing the design of a tubular reactor (1) for gas phase phosgenation of at least one organic amine, the method comprising the steps of:
a. defining a desired residence time for the reaction mixture in the reaction zone of the reactor;
b. calculating a theoretical residence time from the reactor geometry and the envisaged process conditions including temperature, pressure and feed streams of phosgene, amine and optionally inert gas;
c. comparing the desired residence time and the calculated theoretical residence time;
d. adjusting the reactor design until the calculated theoretical residence time falls within the range from 50 % to 200 %, preferably 70 % to 150 %, of the desired residence time.
e. Selecting a nozzle position and/or a sleeve from the adjusted reactor design for assembling the tubular reactor.
wherein the assembled tubular reactor (1) preferably comprises
- at least one inlet device (3a) for starting materials,
- an outer shell (2) with inner diameters Dᵣᵢ,
- a tubular inner sleeve (4) disposed within a segment of the outer shell, having a length Lₛ along its longitudinal axis and outer diameters Dₛₒ,
and wherein preferably along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap (5) between the inner sleeve (4) and the outer shell (2), and wherein the inlet device (3a) is disposed within the outer shell (2).

The invention is illustrated further by reference to the drawing and examples, but without being limited thereto.

### Examples:

### General Procedures:

Gas phase phosgenation reactions were carried out in a cylindrical tubular reactor equipped with an inner sleeve (2 mm wall thickness) and preheated to 320 °C. The reactor was arranged vertically and a coaxial nozzle (amine nozzle) was arranged on the axis of the reactor inside the sleeve with the outlet flow being directed downwards. Amine was evaporated, superheated to 320 °C and fed to the reactor via the amine nozzle. Phosgene was also preheated to 320 °C and fed to the reaction space inside the inner sleeve from the top of the reactor, so that it passed the amine nozzle on its outside. The reaction pressure was set at 300 mbar(g) and the molar phosgene excess was set at 140 %. At the bottom end of the inner sleeve the reaction gases were passed into a quench zone where they were quenched by the addition of Chlorobenzene through a set of spray nozzles arranged at the outer wall of the reactor. In order maintain the same residence times among phosgenation experiments of the same amine while using sleeves of different diameters, the length of the reaction zone between the amine nozzle and the quench was adjusted by positioning the amine nozzle at different heights in the reactor. This facilitates an isolated observation of the effects caused by varying the diameter of the inner sleeve. Effects caused by the associated change of the gas velocity that would otherwise lead to a change in residence times, are mostly eliminated by this approach, because the residence time remains unchanged.

It is noteworthy that the chosen setup and operating conditions were not optimized for long runtimes of the phosgenation reaction. The sole purpose was to isolate and observe the effect of the different diameter ratios of the outer diameter of the inner sleeve to the inner diameter of the outer reactor wall. Therefore, the absolute runtimes cannot be compared to absolute runtimes which might be described in previous literature.

Hydrolyzable chlorine content of the crude product was measured according to ISO 15028:2014.

Chloroalkyl isocyanate contents of the crude isocyanates were determined by gas chromatography.

### Example 1 (comparative)

A tube reactor with an outer shell having an inner diameter of 350 mm was equipped with a cylindric inner sleeve with an outer diameter of 348 mm resulting in a diameter ratio of 0.992. The reaction was carried out as described in the general procedure using hexamethylene diamine as the amine. About 1 day into the continuous reaction, a temperature probe located at the outside wall of the reactor registered temperature fluctuations between 400 °C and 420 °C, before the measured temperature dropped to 358 °C after 45 h. At that time, also an increase of the 6-chlorohexyl isocyanate (CHI) content of the crude product from less than 0.2 wt % to 0.45 wt % was observed and the hydrolyzable chlorine (HC) content of the crude product increased. After 55 hours the run had to be aborted and the reactor was opened for inspection. First observation was that the inner sleeve stuck in the reactor and required more force to pull it out than any of the other tested inner sleeves. Besides that, a larger chunk of solids was found on one side of the inner sleeve. Downstream of it, a bluish discoloration of the reactor sleeve was observed on the entire circumference of the reactor sleeve. It is assumed that the deposit deflected the gas stream and thereby caused the observed temperature fluctuations as well as excessive temperature at the sleeve as indicated by the discoloration.

### Example 2 (comparative)

A tube reactor with an outer shell having an inner diameter of 350 mm was equipped with a cylindric inner sleeve having an outer diameter of 203 mm resulting in a diameter ratio of 0.579. The reaction was carried out as described in the general procedure using hexamethylene diamine as the amine. At about 46 hours into the continuous reaction, the crude product turned turbid with solid particles and the differential pressure over the reaction zone increased. At this time, again, the CHI content of the crude product was almost doubled compared the previous day and also the HC content was increased. After 72 h, the reaction had to be stopped and the reactor was opened for inspection. While the amine nozzle and the upper parts of the inner sleeve showed only light deposits, a large amount of brownish solids was found at the very end of the sleeve and the reactor walls right downstream of the sleeves end. A discoloration of the sleeve as in comparative example 1 was not observed.

### Example 3 (inventive)

A tube reactor with an outer shell having an inner diameter of 350 mm was equipped with a cylindric inner sleeve with an outer diameter of 252 mm resulting in a diameter ratio of 0.720.

The reaction was carried out as described in the general procedure using hexamethylene diamine as the amine with the nozzle being positioned further upstream than in comparative example 2. At about 65 h into the continuous reaction, the crude product turned turbid with solid particles and the differential pressure over the reaction zone increased. However, the CHI content remained below 0.2 wt % and only the HC content of the crude reaction product increased at this point in time. After 87 h, the reaction had to be stopped to a too high differential pressure over the reaction zone and the reactor was opened for inspection. Again, deposits of a brownish solid were found at the very end of the sleeve and the reactor walls right downstream of the sleeves end. A discoloration of the sleeve as in comparative example 1 was not observed.

### Example 4 (inventive)

A tube reactor with an outer shell having an inner diameter of 350 mm was equipped with a cylindric inner sleeve with an outer diameter of 334 mm resulting in a diameter ratio of 0.955. The reaction was carried out as described in the general procedure using hexamethylene diamine as the amine. At 100 h the continuous reaction was still in operation with differential pressure over the reaction zone still being well within operation window. CHI and HC contents of the crude reaction product remained stable and the reaction was terminated. During inspection of the reactor and sleeve, only light deposits were found.

### Example 5 (inventive)

A tube reactor with an outer shell having an inner diameter of 160 mm was equipped with a cylindric inner sleeve with an outer diameter of 114 mm resulting in a diameter ratio of 0.711. The reaction was carried out as described in the general procedure using pentamethylene diamine as the amine. The continuous reaction was operated for 78 h, before it had to be stopped due to a too high differential pressure over the reaction zone. Inspection revealed deposits of a brownish solid at the very end of the sleeve and the reactor walls right downstream of the sleeves end, similar to what was observed in Example 3.

### Example 6 (inventive)

A tube reactor with an outer shell having an inner diameter of 160 mm was equipped with a cylindric inner sleeve with an outer diameter of 139 mm resulting in a diameter ratio of 0.871.

The reaction was carried out as described in the general procedure using pentamethylene diamine as the amine. At 130 h the continuous reaction was still in operation with differential pressure over the reaction zone still being well within operation window. The reaction was terminated and during inspection of the reactor and sleeve, only light deposits were found.

## Claims

1. A tubular reactor (1) for gas phase phosgenation of at least one organic amine comprising
• at least one inlet device (3a) for starting materials,
• an outer shell (2) with inner diameters Dᵣᵢ
• a tubular inner sleeve (4) disposed within a segment of the outer shell, having a length Lₛ along its longitudinal axis and outer diameters Dₛₒ,
wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap (5) between the tubular inner sleeve (4) and the outer shell (2), and wherein the inlet device (3a) is disposed within the outer shell (2).

2. The reactor according to claim 1 wherein R_{d} is in the range from 0.600 to 0.990 along at least 70 % of the length Lₛ of the inner sleeve (4), preferably along at least 90 % of the length Lₛ of the inner sleeve (4), more preferably along at least 95 % of the length Lₛ of the inner sleeve (4) and even more preferably along at least 99 % of the length Lₛ of the inner sleeve (4).

3. The reactor according to claim 1 or 2 wherein the inlet device (3a) for the amine comprises an annular gap nozzle.

4. The reactor according to any of claims 1 to 2 wherein the gap (5) between the inner sleeve and the outer shell (2) has a width that does not deviate more than 30 %, preferably not more than 10 % and more preferably not more than 5 % from its narrowest width within a given cross section perpendicular to the longitudinal axis of the inner sleeve (4).

5. The reactor according to any of claims 1 to 4 wherein at least the inner sleeve (4), preferably the inner sleeve (4) and the segment of the outer shell through which the inner sleeve extends (11), are rotationally symmetric at 120° or at 180°.

6. The reactor according to any of claims 1 to 5 wherein the inner sleeve (4) is of a cylindrical shape over at least 70 % of its length along the gas flow path.

7. The reactor according to any of claims 1 to 6 wherein the ratio R_{d} is in the range from 0.700 to 0.985, preferably in the range from 0.750 to 0.980 and more preferably in the range from 0.800 to 0.975.

8. The reactor according to any of claims 1 to 7 wherein the length Lₛ of the inner sleeve (4) along its longitudinal axis is from 1.5 x its largest diameter Dₛₒ to 150 x its largest Diameter Dₛₒ, preferably from 3 x its largest diameter Dₛₒ to 100 x its largest diameter Dₛₒ, even more preferably 10 x its largest diameter Dₛₒ to 70 x its largest diameter Dₛₒ and most preferably 12 x its largest diameter Dₛₒ to 50 x its largest diameter Dₛₒ.

9. The reactor according to any of claims 1 to 8 wherein the width of the gap (5) that exists between the inner sleeve (4) and the outer shell (2) is in the range from 1 to less than 50 mm, preferably 1 to 49 mm, more preferably 2 to 48 mm, even more preferably 4 to 45 and most preferably 8 to 40 mm.

10. The reactor according to any of claims 1 to 9 further comprising one or more quench nozzles (7) and wherein the length L_{S} of the inner sleeve (4) reaches from top of the sleeve near the inlet devices (3) to the bottom near the quench nozzles (7) in the direction of its longitudinal axis.

11. A process for the production of an isocyanate by phosgenation of at least one organic amine, comprising the steps of
(i) providing a gaseous stream of the amine ;
(ii) providing a gaseous stream of phosgene;
(iii) mixing the amine stream from step (i) and the phosgene stream from step (ii) and passing the resulting mixture through a reaction zone to conduct a reaction of the amine with excess phosgene and obtain a gaseous reaction product mixture;
(iv) cooling the reaction product mixture obtained in step (iii) by contacting it with a quench liquid in a quench zone to obtain a quenched product mixture; and
(v) separating the isocyanate from the quenched product mixture obtained in step (iv)
**characterized in that** the reaction in step (iii) is carried out in a reactor (1) according to any of claims 1 to 10.

12. The process according to claim 11 comprising the additional steps of
(vi) shutting down the phosgenation;
(vii) removing the inner sleeve (4) from the reactor;
(viii) inserting a new or cleaned inner sleeve (4) into the reactor; and
(ix) restarting the phosgenation.

13. The process according to claim 11 or 12 wherein before the phosgenation is started or restarted, the steps of
(x) defining the type of organic amine to be phosgenated and the desired throughput for the following production run and providing this information;
(xi) analyzing the information provided in step (x) to determine a sleeve size and a nozzle position to be used; and
(xii) mounting the sleeve (4) and the amine nozzle as determined in step (xi) into the tubular reactor;
are carried out.

14. The process according to any of claims 11 to 13 wherein the organic amine is selected from the group consisting of hexamethylene diamine, pentamethylene diamine and 1-amino-3,5,5-trimethyl-5-aminomethylcyclohexane.

15. A computer implemented method for optimizing the design of a tubular reactor (1) for gas phase phosgenation of at least one organic amine, the method comprising the steps of:
a. defining a desired residence time for the reaction mixture in the reaction zone of the reactor;
b. calculating a theoretical residence time from the reactor geometry and the envisaged process conditions including temperature, pressure and feed streams of phosgene, amine and optionally inert gas;
c. comparing the desired residence time and the calculated theoretical residence time;
d. adjusting the reactor design until the calculated theoretical residence time falls within the range from 50% to 200%, preferably 70% to 150%, of the desired residence time.
e. Selecting a nozzle position and/or a sleeve from the adjusted reactor design for assembling the tubular reactor.
wherein the assembled tubular reactor (1) comprises
• at least one inlet device (3a) for starting materials,
• an outer shell (2) with inner diameters Dᵣᵢ,
• a tubular inner sleeve (4) disposed within a segment of the outer shell, having a length Lₛ along its longitudinal axis and outer diameters Dₛₒ,
and wherein along at least 50% of length Lₛ, having an inner diameter Dᵣᵢ and an outer diameter Dₛₒ, ratio R_{d} is from 0.600 to 0.990, calculated by the following equation R_{d} = Dₛₒ/Dᵣᵢ, wherein there is a gap (5) between the inner sleeve and the outer shell (2), and wherein the inlet device (3) is disposed within the outer shell (2).
